# EUROPÄISCHE PATENTSCHRIFT

(11) **EP 0 820 257 B1**
(45) Veröffentlichungstag und Bekanntmachung des Hinweises auf die Patenterteilung: **03.11.1999**
(21) Anmeldenummer: 96909018.2
(22) Anmeldetag: 29.03.1996
(51) Int. Cl.: A61B 19/00

(54) **VORRICHTUNG ZUR STIMULATION DER GEWEBSNEUBILDUNG BEI GEWEBSDEFEKTEN**
DEVICE FOR STIMULATING NEW TISSUE FORMATION IN TISSUE DEFECTS
DISPOSITIF POUR STIMULER LA FORMATION DE NOUVEAUX TISSUS LORS DE LESIONS TISSULAIRES

(30) Priorität: 13.04.1995 DE 19513961
(43) Veröffentlichungstag der Anmeldung: 28.01.1998
(73) Patentinhaber: Fleischmann, Wim, 89182 Bernstadt (DE)
(72) Erfinder: Fleischmann, Wim, 89182 Bernstadt (DE)
(74) Vertreter: Patentanwälte Westphal, Mussgnug & Partner
(86) Internationale Anmeldenummer: DE9600608
(87) Internationale Veröffentlichungsnummer: WO9632073

(56) Entgegenhaltungen:
- DE-U- 9 320 582
- FR-A- 2 615 397
- US-A- 2 910 061
- US-A- 4 955 905

## Beschreibung

Die Erfindung betrifft eine Vorrichtung zur Stimulation der Gewebsneubildung bei Gewebsdefekten gemäß dem Oberbegriff des Anspruchs 1.

Zur Beschleunigung des Schließens und Verheilens großflächiger und tiefer Gewebsdefekte wird die Gewebsdistraktion eingesetzt, bei weicher auf die an den Gewebsdefekten angrenzenden Gewebsbereiche eine Zugspannung ausgeübt wird. Es hat sich gezeigt, daß eine solche auf das Gewebe ausgeübte Distraktionskraft zu einer verstärkten Gewebsproliferation und damit zu einem schnelleren Schließen der Wunde führt.

Die Distraktionskraft kann im wesentlichen in der Ebene des Gewebsdefektes durch einen Hautdistraktor eingeleitet werden (z.B. US 5 263 971). Dies hat sich insbesondere bei großflächigen Hautdefekten geringer Tiefe bewährt. Bei Gewebsdefekten größerer Tiefe ist zusätzlich eine Distraktionskraft senkrecht zur Ebene des Defektes vorteilhaft (z.B. DE-GM 93 20 582). Diese vertikale Distraktionskraft kann entweder über eine an dem Hautdistraktor senkrecht angreifende Zugkraft eingeleitet werden oder dadurch, daß die Wunde mittels einer Folie vakuumdicht versiegelt wird und durch eine vertikale Zugkraft ein Unterdruck auf das Gewebe unter der Folie ausgeübt wird.

Der Erfindung liegt die Aufgabe zugrunde, die Gewebsproliferation bei der Gewebsdistraktion weiter zu verbessern.

Diese Aufgabe wird erfindungsgemäß gelöst durch eine Vorrichtung mit den Merkmalen des Anspruchs 1.

Vorteilhafte Ausführungen der Erfindung sind in den Unteransprüchen angegeben.

Der Erfindung liegt die Erkenntnis zugrunde, daß die Gewebsproliferation günstig beeinflußt wird, wenn die in das Gewebe eingeleitete Distraktionskraft nicht konstant bleibt, sondern sich periodisch ändert. Die relative Änderung der eingeleiteten Distraktionskraft liegt dabei im Bereich von einigen wenigen Prozent oder darunter. Die Periodendauer der Änderung der Distraktionskraft liegt dabei im Bereich von wenigen Sekunden oder darunter. Von dieser Erkenntnis ausgehend stellt die Erfindung eine Vorrichtung zur Verfügung, die der Distraktionskraft eine sich periodisch ändernde Zugkraft überlagert. Diese sich periodisch ändernde Zugkraft greift dabei vorzugsweise an den Mitteln an, die zum Ausüben der Distraktionskraft eingesetzt werden, d.h. beispielsweise an einem Hautdistraktor der in den Wundrändern verankert ist, oder an einer die Wunde überdeckenden Vakuumversiegelung.

Zweckmäßigerweise wird die zusätzliche periodische Zugkraft über ein Zugseil eingeleitet, so daß diese Zugkraft unter einem Winkel zur Ebene des Gewebsdefektes wirkt. Je nach der Art der Erzeugung der Distraktionskraft wirkt die periodische Zugkraft somit unter einem Winkel von 0° bis 90° zu der Distraktionskraft.

In einer konstruktiv einfachen und vorteilhaft am Krankenbett installierbaren Ausführung greift ein Zugseil an dem an der Wunde angeordneten Distraktionsmittel an. Das Zugseil wird gespannt gehalten und eine periodisch aus das Zugseil einwirkende Querauslenkung erzeugt die sich periodisch ändernde Zugkraft.

Um bei Positionsverlagerungen des Patienten das Zugseil einerseits gespannt zu halten und andererseits keine unerwünschte zusätzliche Zugkraft zu erzeugen, sind Maßnahmen vorgesehen, damit das Zugseil Bewegungen der an der Wunde angebrachten Distraktionsmittel folgen kann.

In einer Ausführung ist hierzu an dem den Distraktionsmitteln entgegengesetzten Ende des Zugseils ein über eine Umlenkscheibe geführtes Gewicht angebracht. Das Gewicht hält das Zugseil gespannt und folgt Positionsverlagerungen des Patienten durch Auf- und Abbewegungen. Das Gewicht erzeugt dabei in dem Zugseil eine konstante Vorspannung, die als Distraktionskraft oder als Teilkomponente der Distraktionskraft verwendet werden kann. Diese Vorspannung kann durch Änderung des Gewichtes vorgegeben werden.

Damit die periodische Querauslenkung des Zugseiles eine entsprechende periodische Änderung der Zugkraft bewirkt, muß das Ende des Zugseiles durch das Gewicht ausreichend festgehalten werden. Bei größeren Gewichten, d.h. einer hohen Vorspannung des Zugseiles, ist die Massenträgheit des Gewichtes hierfür ausreichend. Bei kleineren Gewichten, die einer geringeren Vorspannung des Zugseiles entsprechen, sind Zusätzliche Dämpfungsmaßnahmen vorteilhaft, damit das Ende des Zugseiles nicht den Querauslenkungen folgen kann. Hierzu kann eine Fluiddämpfung verwendet werden. Ebenso kann das Zugseil zur Dämpfung nach Art einer Bandbremse über die Umlenkscheibe geführt werden.

In einer weiteren Ausführung kann das dem Distraktor entgegengesetzte Ende des Zugseiles an einem festen Gegenlagerpunkt fixiert sein. Das Zugseil wird durch eine Gasdruckfeder gespannt gehalten, wobei die Gasdruckfeder Positionsverlagerungen des Patienten ohne Änderung der Spannung des Zugseiles zuläßt, jedoch eine ausreichende Dämpfung bewirkt, so daß die Querauslenkungen des Zugseiles zu einer variierenden Zugkraft führen.

Im folgenden wird die Erfindung anhand von in der Zeichnung dargestellten Ausführungsbeispielen näher erläutert. Es zeigen :
- Figur 1: eine erste Ausführung der Vorrichtung und
- Figur 2: eine zweite Ausführung der Vorrichtung.

In der Zeichnung sind die Mittel zum Ausüben der Distraktionskraft auf das den Gewebsdefekt umgebende Gewebe nur schematisch dargestellt und mit 10 bezeichnet. Diese Mittel 10 zum Einleiten der Distraktionskraft können ein in dem Wundrand verankerter Hautdistraktor oder eine die Wunde überdeckende Vakuumversiegelung sein.

An dem Distraktionsmittel 10 greift unter einem Winkel zur Ebene des Gewebsdefektes ein Zugseil 12 an. Das Zugseil 12 ist gespannt über eine drehbar gelagerte Umlenkrolle 14 und eine Umlenkscheibe 16 geführt. Zwischen der Umlenkrolle 14 und der Umlenkscheibe 16 liegt eine Exzenterscheibe 18 mit ihrer Umfangsnut an dem Zugseil 12 an. Die Exzenterscheibe 18 wird über einen nicht dargestellten Antrieb mit einer Drehzahl in der Größenordnung von z.B. einer Umdrehung pro Sekunde angetrieben und lenkt dadurch das zwischen der Umlenkrolle 14 und der Umlenkscheibe 16 gespannt gehaltene Zugseil 12 in Querrichtung aus, so daß sich die über das Zugseil 12 auf das Distraktionsmittel 10 ausgeübte Zugkraft periodisch mit der Umdrehung der Exzenterscheibe 18 ändert.

Die über das Zugseil 12 auf das Distraktionsmittel 10 ausgeübte Zugkraft kann mittels einer Zugkraft-Meßeinrichtung 20, z.B. einer in das Zugseil 12 eingesetzten Federwaage, gemessen und überwacht werden.

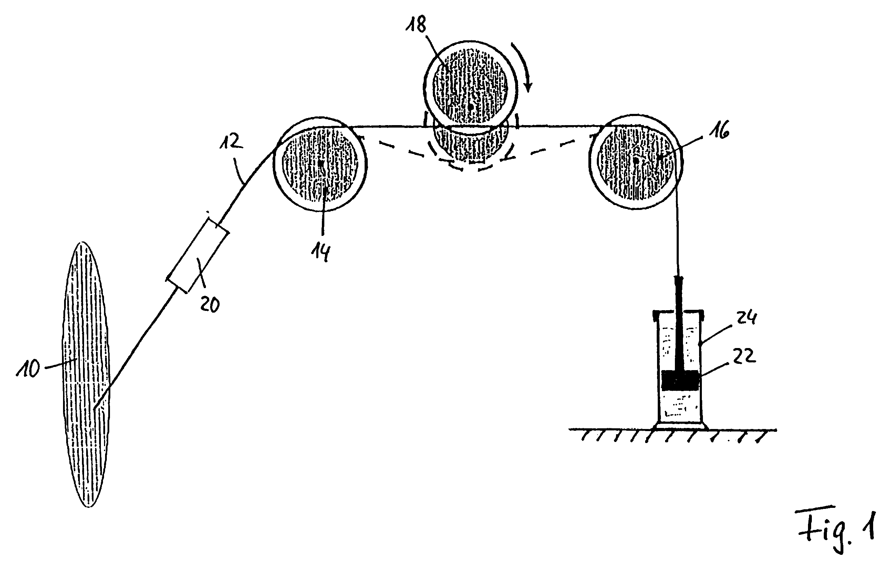

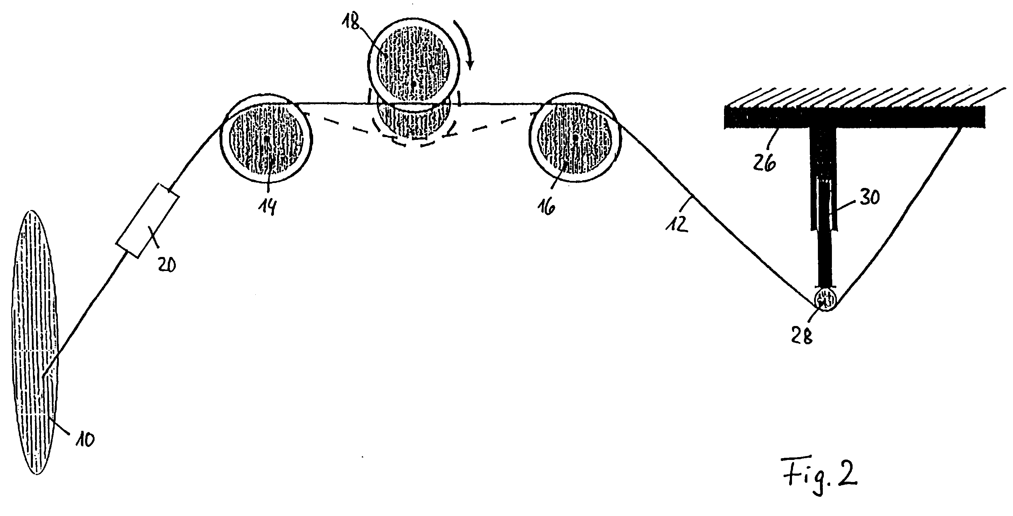

Insoweit stimmen die Ausführungsbeispiele der Figuren 1 und 2 überein. Die Ausführungsbeispiele der Figuren 1 und 2 unterscheiden sich darin, in welcher Weise das Zugseil 12 gespannt gehalten wird.

In dem Ausführungsbeispiel der Figur 1 ist an dem über die drehbar gelagerte Umlenkscheibe 16 geführten freien zweiten Ende des Zugseiles 12 ein Gewicht 22 angebracht. Das Gewicht 22 übt entsprechend seiner Gewichtskraft eine konstante Vorspannung auf das Zugseil 12 aus, die eine konstante Distraktionskraft über das Distraktionsmittel 10 bewirkt. Dieser durch das Gewicht 22 erzeugten Vorspannung wird die periodische Zugkraft überlagert, die durch die Querauslenkung des Zugseiles 12 mittels der rotierenden Exzenterscheibe 18 erzeugt wird.

Bei einer ausreichend großen Massenträgheit des Gewichtes 22 folgt das Gewicht 22 den schnellen Querauslenkungen des Zugseiles 12 durch die Exzenterscheibe 18 nicht, so daß das zweite Ende des Zugseiles 12 durch das Gewicht 22 bezüglich der sich periodisch ändernden Zugkraft quasistationär festgehalten ist.

Ändert der Patient seine Körperposition, so bewegt sich auch das Distraktionsmittel 10, das an der Wunde angebracht ist. Diese Bewegung des Patienten verläuft langsam im Vergleich zu der Periode der durch die Exzenterscheibe 18 bewirkten Zugkraftänderung. Das Gewicht 22 kann dieser durch die Positionsverlagerung des Patienten verursachten langsamen Bewegung folgen, so daß das Zugseil 12 mit konstanter Vorspannung gespannt gehalten wird.

Durch die Wahl des Gewichtes 22 kann eine gewünschte Vorspannung bzw. eine gewünschte konstante Distraktionskraftkomponente gewählt werden.

Um eine Pendelbewegung des Gewichtes 22, z.B. aufgrund der periodisch durch die Exzenterscheibe 18 auf das Zugseil 12 wirkenden Kraft, zu verhindern, kann das Gewicht 22 in einem Zylinder 24 vertikal geführt werden. Dabei kann das Gewicht 22 als Kolben in dem Zylinder 24 ausgebildet sein, so daß sich eine zusätzliche Fluiddämpfung für die Vertikalbewegung des Gewichtes 22 ergibt. Diese Fluiddämpfung verhindert zusätzlich zu der Massenträgheit des Gewichtes 22, daß das Gewicht 22 der Auslenkbewegung des Zugseiles 12 durch die Exzenterscheibe 18 folgt. Eine solche Fluiddämpfung ist dann besonders vorteilhaft, wenn nur ein geringes Gewicht 22 verwendet wird, um dem Zugseil 12 nur eine geringe Vorspannung zu erteilen. Wird der Zylinder 24 mit einer Dämpfungsflüssigkeit gefüllt, so ergibt sich eine besonders starke Dämpfung. Verstellbare Durchtrittsöffnungen des durch das Gewicht 22 gebildeten Kolbens können vorgesehen werden, um die Dämpfung einstellbar auszubilden.

In einer Abwandlung der Ausführung der Figur 1 kann eine Dämpfung der Vertikalbewegung des Gewichtes 22 dadurch erreicht werden, daß die Umlenkscheibe 16 drehfest gehalten wird, so daß das Zugseil 12 nach Art einer Bandbremse unter Reibung über die Umlenkscheibe 16 läuft. Die Reibung des Zugseiles 12 auf dem Umfang der Umlenkscheibe 16 verhindert dabei, daß das freie Ende des Zugseiles 12 mit dem Gewicht 22 den schnellen Querauslenkungen des Zugseiles 12 durch die Exzenterscheibe 18 folgen kann. Das Ausmaß der Abbremsung kann durch eine entsprechende Umfangsbeschichtung der Umlenkscheibe 16 sowie durch eine geeignete Materialwahl des Zugseiles 12 bestimmt und verbessert werden.

Bei dieser Ausführung wirkt sich vorteilhaft aus, daß während der in der Zeichnung gestrichelt eingezeichneten Querauslenkung des Zugseiles 12 sich dieses über einen größeren Winkel an den Umfang der Umlenkscheibe 16 anlegt, so daß der Reibungswiderstand vergrößert wird.

Der Umfang der Umlenkscheibe 16 kann in dieser Ausführung auch nur über einen Teilwinkelbereich mit einer reibungserhöhenden Beschichtung versehen sein. Durch Verdrehen der Umlenkscheibe 16 kann auf diese Weise ein mehr oder weniger großer Winkelbereich der Umlenkscheibe 16, der mit der reibungserhöhenden Beschichtung versehen ist, mit dem Zugseil 12 in Berührung kommen. Dadurch ist der Reibungswiderstand und damit die Dämpfung verstellbar.

In der in Figur 2 dargestellten Ausführung ist das dem Distraktionsmittel 10 entgegengesetzte zweite Ende des Zugseiles 12 fest an einem Rahmen 26 der Vorrichtung befestigt. Zwischen dem Rahmen 26 und der drehbar gelagerten Umlenkscheibe 16 ist das Zugseil 12 über eine Rolle 28 geführt, die an einer an dem Rahmen 26 befestigten Gasdruckfeder 30 gelagert ist. Die Gasdruckfeder 30 hält das Zugseil 12 mit einer durch ihren Federdruck vorgegebenen Vorspannung gespannt.

Bei einer Positionsverlagerung des Patienten kann das Zugseil 12 der Bewegung des Distraktionsmittels 10 folgen, indem die Gasdruckfeder 30 teleskopisch ein- oder ausgefahren wird. Die auf das Zugseil 12 durch die Gasdruckfeder 30 ausgeübte Vorspannung ändert sich dabei aufgrund der Federcharakteristik der Gasdruckfeder 30 nicht.

Die Dämpfung der Gasdruckfeder 30 ist jedoch so groß, daß die Gasdruckfeder 30 den schnellen Querauslenkbewegungen des Zugseiles 12 durch die Exzenterscheibe 18 nicht folgen kann. Bezüglich der periodischen Änderung der Zugkraft durch die Exzenterscheibe 18 wird das Ende des Zugseiles 12 über die Gasdruckfeder 30 somit quasistationär festgehalten.

## Patentansprüche

1. Vorrichtung zur Stimulation der Gewebsneubildung bei Gewebsdefekten mit Distraktionsmitteln zum Ausüben einer Distraktionskraft auf die an den Gewebsdefekt angrenzenden Gewebsbereiche, dadurch gekennzeichnet, daß der Distraktionskraft eine Zugkraft überlagert ist, deren Größe sich periodisch ändert.

2. Vorrichtung nach Anspruch 1, dadurch gekennzeichnet, daß die Zugkraft an den Distraktionsmitteln (10) angreift.

3. Vorrichtung nach Anspruch 2, dadurch gekennzeichnet, daß die Zugkraft über ein Zugseil (12) an den Distraktionsmitteln (10) angreift.

4. Vorrichtung nach Anspruch 3, dadurch gekennzeichnet, daß das Zugseil (12) eine von der periodischen Zugkraft überlagerte Vorspannung aufweist, die zumindest eine Teilkomponente der Distraktionskraft bildet.

5. Vorrichtung nach Anspruch 3 oder 4, dadurch gekennzeichnet, daß das Zugseil (12) zwischen seinem ersten an den Distraktionsmitteln (10) angebrachten Ende und seinem zweiten an einem Gegenlagerpunkt (22, 26) befestigten Ende gespannt ist und daß die Zugkraft durch eine periodische Querauslenkung des Zugseiles (12) bewirkt wird.

6. Vorrichtung nach Anspruch 5, dadurch gekennzeichnet, daß die Querauslenkung durch eine an dem Zugseil (14) angreifende Exzenterscheibe (18) bewirkt wird.

7. Vorrichtung nach Anspruch 3, dadurch gekennzeichnet, daß das Zugseil (12) Bewegungen der Distraktionsmittel (10) ohne Änderung der Zugkraft und gegebenenfalls der Vorspannung folgt.

8. Vorrichtung nach Anspruch 7, dadurch gekennzeichnet, daß die Folgebewegung des Zugseiles (12) so gedämpft ist, daß sie langsamer abläuft als die Periodendauer der Zugkraft.

9. Vorrichtung nach den Ansprüchen 4, 5 und 7, dadurch gekennzeichnet, daß das zweite Ende des Zugseiles (12) an einem über eine Umlenkscheibe (16) geführten Gewicht (22) als Gegenlagerpunkt befestigt ist.

10. Vorrichtung nach den Ansprüchen 8 und 9, dadurch gekennzeichnet, daß die Bewegung des Gewichtes (22) gedämpft ist.

11. Vorrichtung nach Anspruch 10, dadurch gekennzeichnet, daß die Bewegung des Gewichtes (22) durch eine Fluid-Dämpfung (24) gedämpft ist.

12. Vorrichtung nach Anspruch 10, dadurch gekennzeichnet, daß das Zugseil (12) als Bandreibungsdämpfung über die Umlenkscheibe (16) läuft.

13. Vorrichtung nach den Ansprüchen 5, 7 und 8, dadurch gekennzeichnet, daß das zweite Ende des Zugseiles (12) an einem festen Gegenlagerpunkt (26) angebracht ist und daß das Zugseil (12) durch eine Gasdruckfeder (30) gespannt gehalten wird.

14. Vorrichtung nach Anspruch 3, dadurch gekennzeichnet, daß in das Zugseil (12) eine Zugkraft-Meßeinrichtung (20) eingesetzt ist.

## Claims

1. A device for stimulating the new formation of tissue in tissue defects with distraction means for exerting a distraction force on the tissue regions adjacent to the tissue defect,
**characterised in that** onto the distraction force is superimposed a tensile force, the magnitude of which periodically changes.

2. A device according to Claim 1,
**characterised in that** the tensile force acts on the distraction means (10).

3. A device according to Claim 2,
**characterised in that** the tensile force acts via a traction rope (12) on the distraction means (10).

4. A device according to Claim 3,
**characterised in that** the traction rope (12) has a prestressing force superimposed by the periodic tensile force, which forms at least one partial component of the distraction force.

5. A device according to Claim 3 or 4,
**characterised in that** the traction rope (12) is tensioned between its first end mounted at the distraction means (10) and its second end attached to an abutment point (22, 26)
**and in that** the tensile force is effected by a periodical transverse deflection of the traction rope (12).

6. A device according to Claim 5,
**characterised in that** the transverse deflection is effected by an eccentric plate (18) acting on the traction rope (14).

7. A device according to Claim 3,
**characterised in that** the traction rope (12) follows movements of the distraction means (10) without changing the tensile force and, where appropriate, the prestressing.

8. A device according to Claim 7,
**characterised in that** the following movement of the traction rope (12) is dampened so that it takes place more slowly than the period of the tensile force.

9. A device according to Claims 4, 5 and 7,
**characterised in that** the second end of the traction rope (12) is attached to a weight (22) passed over a deflection pulley (16) as an abutment point.

10. A device according to Claims 8 and 9,
**characterised in that** the movement of the weight (22) is dampened.

11. A device according to Claim 10,
**characterised in that** the movement of the weight (22) is dampened by a fluid dampening (24).

12. A device according to Claim 10,
**characterised in that** the traction rope (12) runs as a belt frictional dampening over the deflection pulley (16).

13. A device according to Claims 5, 7 and 8,
**characterised in that** the second end of the traction rope (12) is mounted on a secure abutment point (26) **and in that** the traction rope (12) is kept tensioned by a gas pressure spring (30).

14. A device according to Claim 3,
**characterised in that** a tensile force measuring instrument is inserted into the traction rope (12).

## Revendications

1. Dispositif de stimulation de la néoformation des tissus lors de défauts tissulaires avec des moyens de 〈〈 distraction 〉〉 en vue d'exercer une force de distraction sur les zones de tissu contiguës au défaut tissulaire,
caractérisé en ce que
la force de distraction est superposée à une force de traction dont l'importance est modifiée périodiquement.

2. Dispositif selon la revendication 1,
caractérisé en ce que
la force de traction agit sur les moyens de distraction (10).

3. Dispositif selon la revendication 2,
caractérisé en ce que
la force de traction agit par l'intermédiaire d'un câble de traction (12) sur les moyens de distraction (10).

4. Dispositif selon la revendication 3,
caractérisé en ce que
le câble de traction (12) manifeste une pré-tension augmentée par la force de traction périodique qui forme au moins une composante partielle de la force de distraction.

5. Dispositif selon la revendication 3 ou 4,
caractérisé en ce que
le câble de traction (12) est tendu entre sa première extrémité appliquée sur les moyens de distraction (10) et sa seconde extrémité fixée sur un point de contre positionnement (22, 26) et la force de traction est provoquée par une déviation transversale périodique du câble de traction (12).

6. Dispositif selon la revendication 5,
caractérisé en ce que
la déviation transversale est provoquée par une roue d'excentrique (18) qui agit sur le câble de traction (12).

7. Dispositif selon la revendication 3,
caractérisé en ce que
le câble de traction (12) suit les mouvements du moyen de distraction (10) sans modification de la force de traction et éventuellement de la pré-tension.

8. Dispositif selon la revendication 7,
caractérisé en ce que
le mouvement de suite du câble de traction (12) est amorti de telle sorte qu'il se déroule plus lentement que la durée de période de la force de traction.

9. Dispositif selon les revendications 4, 5 et 7,
caractérisé en ce que
la seconde extrémité du câble de traction (12) est fixée à un poids guidé par une poulie de renvoi (16) en tant que point de contre positionnement.

10. Dispositif selon les revendications 8 et 9,
caractérisé en ce que
le mouvement du poids (22) est amorti.

11. Dispositif selon la revendication 10,
caractérisé en ce que
le mouvement du poids (22) est amorti à l'aide d'un amortissement par fluide.

12. Dispositif selon la revendication 10,
caractérisé en ce que
le câble de traction (12) fonctionne par l'intermédiaire de la poulie de renvoi (16) comme un amortissement par friction d'un ruban.

13. Dispositif selon les revendications 5, 7 et 8,
caractérisé en ce que
la seconde extrémité du câble de traction (12) est appliquée sur un point de contre positionnement (26) fixe, et que le câble de traction (12) est maintenu tendu par un ressort à pression de gaz (30).

14. Dispositif selon la revendication 3,
caractérisé en ce qu'
un dispositif de mesure de la force de traction (20) est mis en oeuvre dans le câble de traction (12).
